(19) Europäisches Patentamt
European Patent Office
Office européen des brevets

(11) **EP 1 939 778 A2**

(12) **EUROPEAN PATENT APPLICATION**

(43) Date of publication:
**02.07.2008 Bulletin 2008/27**

(51) Int Cl.:
**G06F 19/00** $^{(2006.01)}$    **C12Q 1/68** $^{(2006.01)}$

(21) Application number: **07253936.4**

(22) Date of filing: **04.10.2007**

(84) Designated Contracting States:
**DE FR GB**
Designated Extension States:
**AL BA HR MK RS**

(30) Priority: **10.10.2006 US 545962**

(71) Applicant: **Agilent Technologies, Inc.**
**Santa Clara, California 95051 (US)**

(72) Inventors:
• **Kincaid, Robert H.**
**Loveland, CO 80537-0599 (US)**
• **Ghosh, Jayati**
**Loveland, CO 80537-0599 (US)**
• **Curry, Bo U.**
**Loveland, CO 80537-0599 (US)**

(74) Representative: **Dunne, Emma Louise et al**
**Williams Powell**
**Staple Court**
**11 Staple Inn Buildings**
**London**
**WC1V 7QH (GB)**

(54) **Analyzing CGH data to identify aberrations**

(57)    Methods, systems and computer readable media for calling out genetic aberrations. Log ratio noise associated with log ratio signals read from respective probes on at least one array for signals representative of the same chromosomal locations in a test sample of nucleic acids and a reference sample of nucleic acids applied to the at least one array are estimated. Outliers for log ratio values from the reference sample to outliers for log ratio values from the test sample are compared. A copy number of one or more of the chromosomal locations in the test sample is outputted relative to the reference sample for viewing by a user.

```
┌────────────────────────────────┐
│  Calculate spread and mean     │
│  for each calibration array    │─── 502
└────────────────────────────────┘
              │
              ▼
┌────────────────────────────────┐
│  Z-normalize log-ratio data    │─── 504
│  of each calibration array     │
└────────────────────────────────┘
              │
              ▼
┌────────────────────────────────┐
│  Compute R, R' and N           │─── 506
│  for each calibration array    │
└────────────────────────────────┘
              │
              ▼
┌────────────────────────────────┐
│  Compute Rtotal/Ntotal         │─── 508
│  and R'total/Ntotal            │
└────────────────────────────────┘
              │
              ▼
┌────────────────────────────────┐
│  Calculate spread for test     │─── 510
│  sample vs. reference sample   │
│  log ratio values              │
└────────────────────────────────┘
              │
              ▼
┌────────────────────────────────┐
│  Make aberration/anomaly calls │─── 512
│  based on Z-score algorithm    │
└────────────────────────────────┘
```

FIG. 4

**Description**

The present invention relates to a method and system for calling out generic aberrations.

Many genomic and genetic studies are directed to the identification of differences in gene dosage or expression among cell populations for the study and detection of disease. For example, many malignancies involve the gain or loss of DNA sequences (alterations in copy number), sometimes entire chromosomes, that may result in activation of oncogenes or inactivation of tumor suppressor genes. Identification of the genetic events leading to neoplastic transformation and subsequent progression can facilitate efforts to define the biological basis for disease, improve prognostication of therapeutic response, and permit earlier tumor detection. In addition, perinatal genetic problems frequently result from loss or gain of chromosome segments such as trisomy 21 or the micro deletion syndromes. Trisomy of chromosome 13 results in Patau syndrome. Abnormal numbers of sex chromosomes result in various developmental disorders. Thus, methods of prenatal detection of such abnormalities can be helpful in early diagnosis of disease.

Comparative genomic hybridization (CGH) is a technique that is used to evaluate variations in genomic copy number in cells. In one implementation of CGH, genomic DNA is isolated from normal reference cells, as well as from test cells (e.g., tumor cells). The two nucleic acids are differentially labeled and then simultaneously hybridized *in situ* to metaphase chromosomes of a reference cell. Chromosomal regions in the test cells which are at increased or decreased copy number can be identified by detecting regions where the ratio of signal from the two distinguishably labeled nucleic acids is altered. For example, those regions that have been decreased in copy number in the test cells will show relatively lower signal from the test DNA that the reference shows, compared to other regions of the genome. Regions that have been increased in copy number in the test cells will show relatively higher signal from the test DNA.

A recent technology development introduced an oligonucleotide array platform for array based comparative genomic hybridization (aCGH) analyses. Such approaches offer benefits over immobilized chromosome approaches, including a higher resolution, as defined by the ability of the assay to localize chromosomal alterations to specific areas of the genome. For further detailed description regarding aCGH technology, the reader is referred to co-pending Application Serial No. 10/744,495 filed December 22, 2003 and titled "Comparative Genomic Hybridization Assays Using Immobilized Oligonucleotide Features and Compositions for Practicing the Same", which is incorporated herein, in its entirety, by reference thereto.

When processing aCGH data, it is important to determine the noise level on signal data read from an aCGH array as accurately as possible, and hence a measure of the minimum log ratio difference required to make reliable amplification or deletion calls, as an underestimation of noise may result in identification of a signal as indicative of an amplification or deletion when it is, in reality, only due to noise. Conversely, an overestimation of noise may result in discarding a signal that is indicative of a true amplification or deletion, as being determined to be within the range of the noise level that was overestimated. Accordingly, there is a need for solutions for more accurately determining the noise level on signal data read from aCGH arrays in order to improve the accuracy and reliability of amplification and deletion calls made based upon analysis of the signal data after removing assessed noise contributions.

According to a first aspect of the present invention there is provided a method as claimed in claim 1.

According to a second aspect of the present invention there is provided a system as claimed in claim 17.

According to a third aspect of the present invention there is provided a computer readable medium as claimed in claim 21. read from respective probes on at least one array for signals representative of the same chromosomal locations in a test sample of nucleic acids and a reference sample of nucleic acids applied to the at least one array, and comparing outliers for log ratio values from the reference sample to outliers for log ratio values from the test sample. A copy number of one or more of the chromosomal locations in the test sample relative to the reference sample is outputted for viewing by a user.

In at least one embodiment, the comparison of outliers is performed for log ratio values from the reference tissue for data points defined by a window extending along a chromosomal location that the data points correspond to, to the data points from the test sample defined by the window in the same corresponding chromosomal location regarding the test sample data points.

In at least one embodiment, the estimating includes calculating the spread of log ratio noise directly from signals from probes contacted to the test sample and signals from probes contacted to the reference sample.

In at least one embodiment, the estimating includes calculating the spread of log ratio noise from signals from probes on at least one calibration array.

In at least one embodiment, the calculating includes Z-normalizing log ratio signal values from the at least one calibration array, and setting positive and negative Z-cutoff values.

In at least one embodiment, the outliers are identified by Z-normalized values greater than the positive Z-cutoff value and Z-normalized values less than the negative Z-cutoff value.

In at least one embodiment, the spread of log ratio signals read from respective probes for signals representative of respective chromosomal locations in the test sample and reference sample is calculated, and a window is provided that surrounds a subset of the log ratio signal values from the probes contacted with the test sample versus the probes contacted with the reference sample. Overabundance or under-abundance of log ratio values that exceed the positive Z-cutoff value or negative Z-cutoff value, respectively are identified, in comparison to the of log ratio values from the at least one calibration array that exceed the positive Z-cutoff value or negative Z-cutoff value.

In at least one embodiment, it is determined whether a positive copy number difference exists between the test and reference sample from Z-scoring according to:

$$Z(w) = \frac{\left(r - n\frac{R}{N}\right)}{\sqrt{n\left(\frac{R}{N}\right)\left(1 - \frac{R}{N}\right)\left(1 - \frac{n-1}{N-1}\right)}}$$

where

Z(w) = the Z-score of log ratio values contained within window w;
R = the number of outliers in the at least one calibration array that exceed the positive cutoff threshold;
N = the total number of log ratio values considered from the at least one calibration array;
r = the number of outliers in the window w that exceed the positive cutoff threshold; and
n = the total number of log ratio values within window w.

In at least one embodiment, the Z-scores are plotted.

In at least one embodiment, calculation includes Z-normalizing log ratio signal values from the probes contacted to said test and reference samples; calculating the derivatives of the Z-normalized log ratio signal values; and setting positive and negative Z-cutoff values.

In at least one embodiment, the outliers are identified by Z-normalized derivative log ratio values greater than the positive Z-cutoff value and Z-normalized values less than the negative Z-cutoff value.

In at least one embodiment, the outliers are identified by pairs of consecutive Z-normalized derivative log ratio values greater than the positive Z-cutoff value or less than the negative Z-cutoff value.

In at least one embodiment, a window is provided that surrounds a subset of the log ratio signal values from the probes contacted with the test sample versus the probes contacted with the reference sample. Overabundance or under-abundance of log ratio values that exceed the positive Z-cutoff value or negative Z-cutoff value, respectively are identified, and the overabundance or under-abundance as a percentage of the total number of log ratio signal values within the window are compared to the number of outliers identified as a percentage of the total number of log ratio signal values.

These and other features of the invention will become apparent to those persons skilled in the art upon reading the details of the methods, systems and computer readable media as more fully described below.

Preferred embodiments of the invention are now described by way of example only and with reference to the drawings in which:

Fig. 1 is a schematic representation of an array.
Fig. 2 is an enlarged view of a portion of the array schematically shown in Fig. 1.
Fig. 3A illustrates a plot of log ratio values between two channels from a CGH array.
Fig. 3B illustrates a plot of the derivatives of the log ratio values plotted in Fig. 3A.
Fig. 3C illustrates the phenomenon that has been observed, that noise levels for probes representative of chromosome locations where amplification or deletion has occurred have been observed to be higher than noise levels of probes representative of chromosome locations that are normal (i.e., no amplification or deletion occurring).
Fig. 3D is a display of charts comparing noise levels among diploid cell lines having been plotted, with noise levels associated with aneuploid cell lines having been plotted, and tumor cell lines.
Fig. 4 are events that may be carried out according to one embodiment of the present invention for making aberration/anomaly calls.
Fig. 5 shows CGH data plotted for Chromosome 17 (Cell Line BT474) 610 relative to the positions on the chromosome 17 shown which the data characterizes.
Fig. 6 illustrates events that may be carried out according to one embodiment of the present invention when no calibration array is used.
Fig. 7A schematically illustrates a plot of log ratio values of test sample signals to reference sample signals against the relative chromosomal positions represented by the signals received.
Fig. 7B shows a plot of the DLR values for the log ratio values plotted in Fig. 7A.
Fig. 8A shows a plot of a distribution of observed log (i.e., $\log_2$) ratio values from autosomes of an individual male/female assay (considered to have no chromosomal aberrations) compared with a plot of a reference normal distribution.

Fig. 8B is a probability plot of the observed log ratio values from Fig. 8A versus Gaussian expectations (i.e., the values that are plotted in the Gaussian distribution of Fig. 7A).

Fig. 8C shows another probability plot of observed log ratio values from a different array from that associated with Figs. 8A and 8B, versus Gaussian expectations.

Fig. 9A shows a probability plot of the log ratio values from Fig. 8A versus Gaussian expectations, and thus corresponds to the plot of Fig. 8B except for the statistical processing option where R, R' and N are computed from the derivative of the log ratio (dLR) (after proper normalization) was used to reduce the number of false positives.

Fig. 9B similarly plots a probability plot of observed log ratio values from a sample containing tissue in the ht29 cancer cell line with normal tissue in the reference channel, versus Gaussian expectations, when using the same statistical processing used in Fig. 9A.

Fig. 9C is a plot similar to the plots shown in figs. 9A and 9B, but where the sample array contained a very aberrant tumor tissue (i.e., a gastric tumor from VTT).

Fig. 10 is a schematic illustration of a typical computer system that may be used to perform method steps described herein.

Before the present systems, methods and computer readable media are described, it is to be understood that this invention is not limited to particular arrays, datasets, software or hardware described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a probe" includes a plurality of such probes and reference to "the array" includes reference to one or more arrays and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

A chemical "array", unless a contrary intention appears, includes any one, two or three-dimensional arrangement of addressable regions bearing a particular chemical moiety or moieties (for example, biopolymers such as polynucleotide sequences) associated with that region, where the chemical moiety or moieties are immobilized on the surface in that region. By "immobilized" is meant that the moiety or moieties are stably associated with the substrate surface in the region, such that they do not separate from the region under conditions of using the array, e.g., hybridization and washing and stripping conditions. As is known in the art, the moiety or moieties may be covalently or non-covalently bound to the surface in the region. For example, each region may extend into a third dimension in the case where the substrate is porous while not having any substantial third dimension measurement (thickness) in the case where the substrate is non-porous. An array may contain more than ten, more than one hundred, more than one thousand more than ten thousand features, or even more than one hundred thousand features, in an area of less than 20 cm$^2$ or even less than 10 cm$^2$. For example, features may have widths (that is, diameter, for a round spot) in the range of from about 10 $\mu$m to about 1.0 cm. In other embodiments each feature may have a width in the range of about 1.0 $\mu$m to about 1.0 mm, such as from about 5.0 $\mu$m to about 500 $\mu$m, and including from about 10 $\mu$m to about 200 $\mu$m. Non-round features may have area ranges equivalent to that of circular features with the foregoing width (diameter) ranges. A given feature is made up of chemical moieties, e.g., nucleic acids, that bind to (e.g., hybridize to) the same target (e.g., target nucleic acid), such that a given feature corresponds to a particular target. At least some, or all, of the features are of different compositions (for example, when any repeats of each feature composition are excluded the remaining features may account for at least 5%, 10%, or 20% of the total number of features). Interfeature areas will typically (but not essentially) be present which do not carry any polynucleotide. Such interfeature areas typically will be present where the arrays are formed by processes involving drop deposition of reagents but may not be present when, for example, light directed synthesis fabrication processes are used. It will be appreciated though, that the interfeature areas, when present, could be of various sizes and configurations. An array is "addressable" in that it has multiple regions (sometimes referenced as "features" or "spots" of the array) of different moieties (for example, different polynucleotide sequences) such that a region at a particular predetermined location (an "address") on the array will detect a particular target or class of targets (although a feature may incidentally detect non-targets of that feature). The target for which each feature is specific is, in representative embodiments, known. An array feature is generally homogenous in composition and concentration and the features may be separated by intervening spaces (although arrays without such separation can be fabricated).

In the case of an array, the "target" will be referenced as a moiety in a mobile phase (typically fluid), to be detected by probes ("target probes") which are bound to the substrate at the various regions. However, either of the "target" or "target probes" may be the one which is to be detected by the other (thus, either one could be an unknown mixture of polynucleotides to be detected by binding with the other). "Addressable sets of probes" and analogous terms refer to the multiple regions of different moieties supported by or intended to be supported by the array surface.

The term "sample" as used herein relates to a material or mixture of materials, containing one or more components of interest. Samples include, but are not limited to, samples obtained from an organism or from the environment (e.g., a soil sample, water sample, etc.) and may be directly obtained from a source (e.g., such as a biopsy or from a tumor) or indirectly obtained e.g., after culturing and/or one or more processing steps. In one embodiment, samples are a complex mixture of molecules, e.g., comprising at least about 50 different molecules, at least about 100 different molecules, at least about 200 different molecules, at least about 500 different molecules, at least about 1000 different molecules, at least about 5000 different molecules, at least about 10,000 molecules, etc.

A "test sample" as applied to CGH analysis, refers to a sample that is being analyzed to evaluate DNA copy number, for example, to look for the presence of genetic anomalies, or species differences, for example. A "reference sample" as applied to CGH analysis, is a sample (e.g., a cell or tissue sample) of the same type as the test sample, but whose quantity or degree of representation is unknown or sequence identity is known. As used herein, a "reference nucleic acid sample" or "reference nucleic acids" refers to nucleic acids comprising sequences whose quantity or degree of representation (e.g., copy number) or sequence identity is known. Similarly, "reference genomic acids" or a "reference genomic sample" refers to genomic nucleic acids comprising sequences whose quantity or degree of representation (e.g., copy number) or sequence identity is known. A "reference nucleic acid sample" may be derived independently from a "test nucleic acid sample", i.e., the samples can be obtained from different organisms or different cell populations of the sample organism. However, in certain embodiments, a reference nucleic acid is present in a "test nucleic acid sample" which comprises one or more sequences whose quantity or identity or degree of representation in the sample is unknown while containing one or more sequences (the reference sequences) whose quantity or identity or degree of representation in the sample is known. The reference nucleic acid may be naturally present in a sample (e.g., present in the cell from which the sample was obtained) or may be added to or spiked into the sample.

A test sample and reference sample may both be contacted to a single array for co-hybridization therewith, wherein log ratios of signals from the two samples can be generated by reading the signals for the test sample on a first channel and reading signals for the reference sample on a two-channel analyzer. Alternatively, the test sample may be hybridized to a first array and the reference sample may be hybridized to a second array that is the same as the first array, and signals from each array may be read, and then compared as log ratios.

An "outlier region" refers to a region of values that is above or below a predefined threshold. Thus, for a predefined upper threshold value, an outlier region lies above the upper threshold value. For a predefined lower threshold value an outlier region lies below the lower threshold value.

An "outlier" is a value that is above or below a predefined threshold, depending upon whether the threshold is an upper threshold value or lower threshold value, respectively.

A "calibration array" refers to an array prepared with a normal malefemale sample with no genetic abnormalities and hence no aberrations along the genome.

The term "genome" refers to all nucleic acid sequences (coding and non-coding) and elements present in any virus, single cell (prokaryote and eukaryote) or each cell type in a metazoan organism. The term genome also applies to any naturally occurring or induced variation of these sequences that may be present in a mutant or disease variant of any virus or cell type. These sequences include, but are not limited to, those involved in the maintenance, replication, segregation, and higher order structures (e.g. folding and compaction of DNA in chromatin and chromosomes), or other functions, if any, of the nucleic acids as well as all the coding regions and their corresponding regulatory elements needed to produce and maintain each particle, cell or cell type in a given organism.

For example, the human genome consists of approximately $3.0 \times 10^9$ base pairs of DNA organized into distinct chromosomes. The genome of a normal diploid somatic human cell consists of 22 pairs of autosomes (chromosomes 1 to 22) and either chromosomes X and Y (males) or a pair of chromosome X's (female) for a total of 46 chromosomes. A genome of a cancer cell may contain variable numbers of each chromosome in addition to deletions, rearrangements and amplification of any subchromosomal region or DNA sequence. In certain aspects, a "genome" refers to nuclear nucleic acids, excluding mitochondrial nucleic acids; however, in other aspects, the term does not exclude mitochondrial nucleic acids. In still other aspects, the "mitochondrial genome" is used to refer specifically to nucleic acids found in mitochondrial fractions.

By "genomic source" is meant the initial nucleic acids that are used as the original nucleic acid source from which the probe nucleic acids are produced, e.g., as a template in the nucleic acid amplification and/or labeling protocols.

If a surface-bound polynucleotide or probe "corresponds to" a chromosomal region, the polynucleotide usually contains a sequence of nucleic acids that is unique to that chromosomal region. Accordingly, a surface-bound polynucleotide that corresponds to a particular chromosomal region usually specifically hybridizes to a labeled nucleic acid made from that chromosomal region, relative to labeled nucleic acids made from other chromosomal regions.

An "array layout" or "array characteristics", refers to one or more physical, chemical or biological characteristics of the array, such as positioning of some or all the features within the array and on a substrate, one or more feature dimensions, or some indication of an identity or function (for example, chemical or biological) of a moiety at a given location, or how the array should be handled (for example, conditions under which the array is exposed to a sample, or array reading specifications or controls following sample exposure).

The phrase " oligonucleotide bound to a surface of a solid support" or "probe bound to a solid support" or a "target bound to a solid support" refers to an oligonucleotide or mimetic thereof, e.g., PNA, LNA or UNA molecule that is immobilized on a surface of a solid substrate, where the substrate can have a variety of configurations, e.g., a sheet, bead, particle, slide, wafer, web, fiber, tube, capillary, microfluidic channel or reservoir, or other structure. In certain embodiments, the collections of oligonucleotide elements employed herein are present on a surface of the same planar support, e.g., in the form of an array. It should be understood that the terms "probe" and "target" are relative terms and that a molecule considered as a probe in certain assays may function as a target in other assays.

As used herein, a "test nucleic acid sample" or "test nucleic acids" refer to nucleic acids comprising sequences whose quantity or degree of representation (e.g., copy number) or sequence identity is being assayed. Similarly, "test genomic acids" or a "test genomic sample" refers to genomic nucleic acids comprising sequences whose quantity or degree of representation (e.g., copy number) or sequence identity is being assayed.

As used herein, a "reference nucleic acid sample" or "reference nucleic acids" refers to nucleic acids comprising sequences whose quantity or degree of representation (e.g., copy number) or sequence identity is known. Similarly, "reference genomic acids" or a "reference genomic sample" refers to genomic nucleic acids comprising sequences whose quantity or degree of representation (e.g., copy number) or sequence identity is known. A "reference nucleic acid sample" may be derived independently from a "test nucleic acid sample," i.e., the samples can be obtained from different organisms or different cell populations of the sample organism. However, in certain embodiments, a reference nucleic acid is present in a "test nucleic acid sample" which comprises one or more sequences whose quantity or identity or degree of representation in the sample is unknown while containing one or more sequences (the reference sequences) whose quantity or identity or degree of representation in the sample is known. The reference nucleic acid may be naturally present in a sample (e.g., present in the cell from which the sample was obtained) or may be added to or spiked in the sample.

If a surface-bound polynucleotide or probe "corresponds to" a chromosome, the polynucleotide usually contains a sequence of nucleic acids that is unique to that chromosome. Accordingly, a surface-bound polynucleotide that corresponds to a particular chromosome usually specifically hybridizes to a labeled nucleic acid made from that chromosome, relative to labeled nucleic acids made from other chromosomes. Array features, because they usually contain surface-bound polynucleotides, can also correspond to a chromosome.

A "non-cellular chromosome composition" is a composition of chromosomes synthesized by mixing pre-determined amounts of individual chromosomes. These synthetic compositions can include selected concentrations and ratios of chromosomes that do not naturally occur in a cell, including any cell grown in tissue culture. Non-cellular chromosome compositions may contain more than an entire complement of chromosomes from a cell, and, as such, may include extra copies of one or more chromosomes from that cell. Non-cellular chromosome compositions may also contain less than the entire complement of chromosomes from a cell.

"CGH" or "Comparative Genomic Hybridization" refers generally to techniques for identification of chromosomal alterations (such as in cancer cells, for example). Using CGH, ratios between tumor or test sample and normal or control sample enable the detection of chromosomal amplifications and deletions of regions that may include oncogenes and tumor suppressive genes, for example.

A "CGH array" or "aCGH array" refers to an array that can be used to compare DNA samples for relative differences in copy number. In general, an aCGH array can be used in any assay in which it is desirable to scan a genome with a sample of nucleic acids. For example, an aCGH array can be used in location analysis as described in U.S. Patent No. 6,410,243, the entirety of which is incorporated herein. In certain aspects, a CGH array provides probes for screening or scanning a genome of an organism and comprises probes from a plurality of regions of the genome. In one aspect, the array comprises probe sequences for scanning an entire chromosome arm, wherein probes targets are separated by at least about 500 bp, at least about 1 kb, at least about 5 kb, at least about 10 kb, at least about 25 kb, at least about 50 kb, at least about 100 kb, at least about 250 kb, at least about 500 kb and at least about 1 Mb. In another aspect, the array comprises probes sequences for scanning an entire chromosome, a set of chromosomes, or the complete complement of chromosomes forming the organism's genome. By "resolution" is meant the spacing on the genome between sequences found in the probes on the array. In some embodiments (e.g., using a large number of probes of high complexity) all sequences in the genome can be present in the array. The spacing between different locations of the genome that are represented in the probes may also vary, and may be uniform, such that the spacing is substantially the same between sampled regions, or non-uniform, as desired. An assay performed at low resolution on one array, e.g., comprising probe targets separated by larger distances, may be repeated at higher resolution on another array, e.g., comprising probe targets separated by smaller distances.

In certain aspects, in constructing the arrays, both coding and non-coding genomic regions are included as probes, whereby "coding region" refers to a region comprising one or more exons that is transcribed into an mRNA product and from there translated into a protein product, while by non-coding region is meant any sequences outside of the exon regions, where such regions may include regulatory sequences, e.g., promoters, enhancers, untranslated but transcribed regions, introns, origins of replication, telomeres, etc. In certain embodiments, one can have at least some of the probes directed to non-coding regions and others directed to coding regions. In certain embodiments, one can have all of the probes directed to non-coding sequences. In certain embodiments, one can have all of the probes directed to coding sequences. In certain other aspects, individual probes comprise sequences that do not normally occur together, e.g., to detect gene rearrangements, for example.

In some embodiments, at least 5 % of the polynucleotide probes on the solid support hybridize to regulatory regions of a nucleotide sample of interest while other embodiments may have at least 30% of the polynucleotide probes on the solid support hybridize to exonic regions of a nucleotide sample of interest. In yet other embodiments, at least 50% of the polynucleotide probes on the solid support hybridize to intergenic (e.g., non-coding) regions of a nucleotide sample of interest. In certain aspects, probes on the array represent random selection of genomic sequences (e.g., both coding and noncoding). However, in other aspects, particular regions of the genome are selected for representation on the array, e.g., such as CpG islands, genes belonging to particular pathways of interest or whose expression and/or copy number are associated with particular physiological responses of interest (e.g., disease, such a cancer, drug resistance, toxological responses and the like). In certain aspects, where particular genes are identified as being of interest, intergenic regions proximal to those genes are included on the array along with, optionally, all or portions of the coding sequence corresponding to the genes. In one aspect, at least about 100 bp, 500 bp, 1,000 bp, 5,000 bp, 10,000 kb or even 100,000 kb of genomic DNA upstream of a transcriptional start site is represented on the array in discrete or overlapping sequence probes. In certain aspects, at least one probe sequence comprises a motif sequence to which a protein of interest (e.g., such as a transcription factor) is known or suspected to bind.

In certain aspects, repetitive sequences are excluded as probes on the arrays. However, in another aspect, repetitive sequences are included.

The choice of nucleic acids to use as probes may be influenced by prior knowledge of the association of a particular chromosome or chromosomal region with certain disease conditions. International Application WO 93/18186 provides a list of exemplary chromosomal abnormalities and associated diseases, which are described in the scientific literature. Alternatively, whole genome screening to identify new regions subject to frequent changes in copy number can be performed using the methods of the present invention discussed further below.

In some embodiments, previously identified regions from a particular chromosomal region of interest are used as probes. In certain embodiments, the array can include probes which "tile" a particular region (e.g., which have been identified in a previous assay or from a genetic analysis of linkage), by which is meant that the probes correspond to a region of interest as well as genomic sequences found at defined intervals on either side, i.e., 5' and 3' of, the region of interest, where the intervals may or may not be uniform, and may be tailored with respect to the particular region of interest and the assay objective. In other words, the tiling density may be tailored based on the particular region of interest and the assay objective. Such "tiled" arrays and assays employing the same are useful in a number of applications, including applications where one identifies a region of interest at a first resolution, and then uses tiled array tailored to the initially identified region to further assay the region at a higher resolution, e.g., in an iterative protocol.

In certain aspects, the array includes probes to sequences associated with diseases associated with chromosomal imbalances for prenatal testing. For example, in one aspect, the array comprises probes complementary to all or a portion of chromosome 21 (e.g., Down's syndrome), all or a portion of the X chromosome (e.g., to detect an X chromosome deficiency as in Turner's Syndrome) and/or all or a portion of the Y chromosome Klinefelter Syndrome (to detect duplication of an X chromosome and the presence of a Y chromosome), all or a portion of chromosome 7 (e.g., to detect William's Syndrome), all or a portion of chromosome 8 (e.g., to detect Langer-Giedon Syndrome), all or a portion of chromosome 15 (e.g., to detect Prader-Willi or Angelman's Syndrome, all or a portion of chromosome 22 (e.g.,

to detect Di George's syndrome).

Other "themed" arrays may be fabricated, for example, arrays including whose duplications or deletions are associated with specific types of cancer (e.g., breast cancer, prostate cancer and the like). The selection of such arrays may be based on patient information such as familial inheritance of particular genetic abnormalities. In certain aspects, an array for scanning an entire genome is first contacted with a sample and then a higher-resolution array is selected based on the results of such scanning.

Themed arrays also can be fabricated for use in gene expression assays, for example, to detect expression of genes involved in selected pathways of interest, or genes associated with particular diseases of interest.

In one embodiment, a plurality of probes on the array are selected to have a duplex $T_m$ within a predetermined range. For example, in one aspect, at least about 50% of the probes have a duplex $T_m$ within a temperature range of about 75°C to about 85°C. In one embodiment, at least 80% of said polynucleotide probes have a duplex $T_m$ within a temperature range of about 75°C to about 85°C, within a range of about 77°C to about 83°C, within a range of from about 78°C to about 82°C or within a range from about 79°C to about 82°C. In one aspect, at least about 50% of probes on an array have range of $T_m$'s of less than about 4°C, less then about 3°C, or even less than about 2°C, e.g., less than about 1.5°C, less than about 1.0°C or about 0.5°C.

The probes on the microarray, in certain embodiments have a nucleotide length in the range of at least 30 nucleotides to 200 nucleotides, or in the range of at least about 30 to about 150 nucleotides. In other embodiments, at least about 50% of the polynucleotide probes on the solid support have the same nucleotide length, and that length may be about 60 nucleotides.

In certain aspects, longer polynucleotides may be used as probes. In addition to the oligonucleotide probes described above, cDNAs, or inserts from phage BACs (bacterial artificial chromosomes) or plasmid clones, can be arrayed. Probes may therefore also range from about 201-5000 bases in length, from about 5001-50,000 bases in length, or from about 50,001-200,000 bases in length, depending on the platform used. If other polynucleotide features are present on a subject array, they may be interspersed with, or in a separately-hybridizable part of the array from the subject oligonucleotides.

In still other aspects, probes on the array comprise at least coding sequences.

In one aspect, probes represent sequences from an organism such as *Drosophila melanogaster, Caenorhabditis elegans,* yeast, zebrafish, a mouse, a rat, a domestic animal, a companion animal, a primate, a human, etc. In certain aspects, probes representing sequences from different organisms are provided on a single substrate, e.g., on a plurality of different arrays.

A "CGH assay" using an aCGH array can be generally performed as follows. In one embodiment, a population of nucleic acids contacted with an aCGH array comprises at least two sets of nucleic acid populations, which can be derived from different sample sources. For example, in one aspect, a target population contacted with the array comprises a set of target molecules from a reference sample and from a test sample. In one aspect, the reference sample is from an organism having a known genotype and/or phenotype, while the test sample has an unknown genotype and/or phenotype or a genotype and/or phenotype that is known and is different from that of the reference sample. For example, in one aspect, the reference sample is from a healthy patient while the test sample is from a patient suspected of having cancer or known to have cancer.

In one embodiment, a target population being contacted to an array in a given assay comprises at least two sets of target populations that are differentially labeled (e.g., by spectrally distinguishable labels). In one aspect, control target molecules in a target population are also provided as two sets, e.g., a first set labeled with a first label and a second set labeled with a second label corresponding to first and second labels being used to label reference and test target molecules, respectively.

In one aspect, the control target molecules in a population are present at a level comparable to a haploid amount of a gene represented in the target population. In another aspect, the control target molecules are present at a level comparable to a diploid amount of a gene. In still another aspect, the control target molecules are present at a level that is different from a haploid or diploid amount of a gene represented in the target population. The relative proportions of complexes formed labeled with the first label vs. the second label can be used to evaluate relative copy numbers of targets found in the two samples.

In certain aspects, test and reference populations of nucleic acids may be applied separately to separate but identical arrays (e.g., having identical probe molecules) and the signals from each array can be compared to determine relative copy numbers of the nucleic acids in the test and reference populations.

Methods to fabricate arrays are described in detail in US Patent 6,242,266; 6,232,072; 6,180,351; 6,171,797 and 6,323,043. As already mentioned, these references are incorporated herein by reference. Other drop deposition methods can be used for fabrication, as previously described herein. Also, instead of drop deposition methods, photolithographic array fabrication methods may be used. Interfeature areas need not be present particularly when the arrays are made by photolithographic methods as described in those patents.

Following receipt by a user, an array will typically be exposed to a sample and then read. Reading of an array may be accomplished by illuminating the array and reading the location and intensity of resulting fluorescence at multiple regions on each feature of the array. For example, a scanner may be used for this purpose is the AGILENT MICROARRAY SCANNER manufactured by Agilent Technologies, Palo, Alto, CA or other similar scanner. Other suitable apparatus and methods are described in US Patents 6,518,556; 6,486,457; 6,406,849; 6,371,370; 6,355,921; 6,320,196; 6,251,685 and 6,222,664. Scanning typically produces a scanned image of the array which may be directly inputted to a feature extraction system for direct processing and/or saved in a computer storage device for subsequent processing. However, arrays may be read by any other methods or apparatus than the foregoing; other reading methods including other optical techniques or electrical techniques (where each feature is provided with an electrode to detect bonding at that feature in a manner disclosed in US Patents 6,251,685, 6,221,583 and elsewhere).

An array is "addressable" when it has multiple regions of different moieties, i.e., features (e.g., each made up of different oligonucleotide sequences) such that a region (i.e., a "feature" or "spot" of the array) at a particular predetermined location (i.e., an "address") on the array will detect a particular solution phase nucleic acid sequence. Array features are typically, but need not be, separated by intervening spaces.

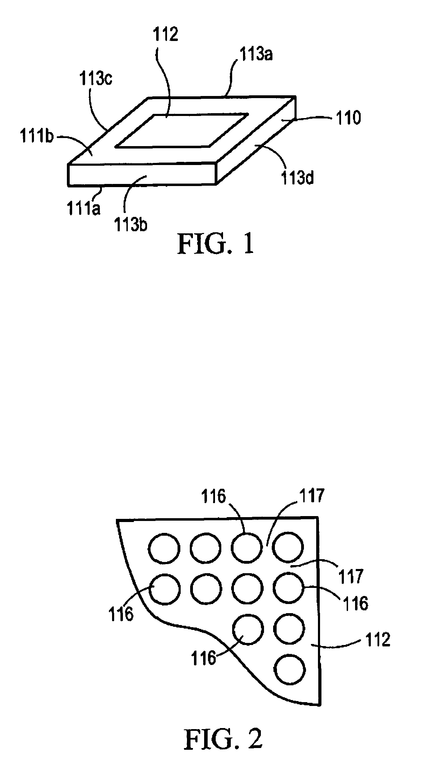

An exemplary array is shown in Figs. 1-2, where the array shown in this representative embodiment includes a contiguous planar substrate 110 carrying an array 112 disposed on a surface 111b of substrate 110. It will be appreciated though, that more than one array (any of which are the same or different) may be present on surface 111b, with or without spacing between such arrays. That is, any given substrate may carry one, two, four or more arrays disposed on a surface of the substrate and depending on the use of the array, any or all of the arrays may be the same or different from one another and each may contain multiple spots or features. The one or more arrays 112 usually cover only a portion of the surface 111b, with regions of the surface 111b adjacent the opposed sides 113c, 113d and leading end 113a and trailing end 113b of slide 110, not being covered by any array 112. A surface 111a of the slide 110 does not carry any arrays 112. Each array 112 can be designed for testing against any type of sample, whether a trial sample, reference sample, a combination of them, or a known mixture of biopolymers such as polynucleotides. Substrate 110 may be of any shape, as mentioned above.

As mentioned above, array 112 contains multiple spots or features 116 of oligomers, e.g., in the form of polynucleotides, and specifically oligonucleotides. As mentioned above, all of the features 116 may be different, or some or all could be the same. The interfeature areas 117 could be of various sizes and configurations. Each feature carries a predetermined oligomer such as a predetermined polynucleotide (which includes the possibility of mixtures of polynucleotides). It will be understood that there may be a linker molecule (not shown) of any known types between the surface 111b and the first nucleotide.

Substrate 110 may carry on surface 111a or elsewhere, an identification code, e.g., in the form of bar code (not shown) or the like printed on a substrate in the form of a paper or plastic label attached by adhesive or any convenient means. The identification code contains information relating to array 112, where such information may include, but is not limited to, an identification of array 112, i.e., layout information relating to the array(s), etc.

In the case of an array in the context of the present application, the "target" may be referenced as a moiety in a mobile phase (typically fluid), to be detected by "probes" which are bound to the substrate at the various regions.

A "scan region" refers to a contiguous (preferably, rectangular) area in which the array spots or features of interest, as defined above, are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

A "design file" is typically provided by an array manufacturer and is a file that embodies all the information that the array designer from the array manufacturer considered to be pertinent to array interpretation. For example, Agilent Technologies supplies its array users with a design file written in the XML language that describes the geometry as well as the biological content of a particular array.

A "design pattern" is a description of relative placement of features, with annotation. A grid template or design pattern can be generated from parsing a design file and can be saved/stored on a computer storage device. A grid template has basic grid information from the design file that it was generated from, which information may include, for example, the number of rows in the array from which the grid template was generated, the number of columns in the array from which the grid template was generated, column spacings, subgrid row and column numbers, if applicable, spacings between subgrids, number of arrays/hybridizations on a slide, etc. An alternative way of creating a grid template is by using an interactive grid mode provided by the system, which also provides the ability to add further information, for example, such as subgrid relative spacings, rotation and skew information, etc.

A "property" of an array, as used herein refers to a characteristic of an array that may be measured through analysis and calculation based on signals received during reading (e.g., scanning or other method of obtaining signals from) the array, and which may be used as a measure of quality of the array. Properties include, but are not limited to, noise, signal-to noise, background signal, signal intensity, uniformity/non-uniformity, population outlier, saturated feature, etc.

A "probe signal", "probe value" or "probe signal value" refers to the ratio of a signal obtained from the probe to the signal of a target hybridized thereto, i.e., the signal from a probe bound to a target.

When one item is indicated as being "remote" from another, this is referenced that the two items are not at the same physical location, e.g., the items are at least in different buildings, and may be at least one mile, ten miles, or at least one hundred miles apart.

"Communicating" information references transmitting the data representing that information as electrical signals over a suitable communication channel (for example, a private or public network).

"Forwarding" an item refers to any means of getting that item from one location to the next, whether by physically transporting that item or otherwise (where that is possible) and includes, at least in the case of data, physically transporting a medium carrying the data or communicating the data.

A "processor" references any hardware and/or software combination which will perform the functions required of it. For example, any processor herein may be a programmable digital microprocessor such as available in the form of a mainframe, server, or personal computer. Where the processor is programmable, suitable programming can be communicated from a remote location to the processor, or previously saved in a computer program product. For example, a magnetic or optical disk may carry the programming, and can be read by a suitable disk reader communicating with each processor at its corresponding station.

Reference to a singular item, includes the possibility that there are plural of the same items present.

"May" means optionally.

Methods recited herein may be carried out in any order of the recited events which is logically possible, as well as the recited order of events.

All patents and other references cited in this application, are incorporated into this application by reference except insofar as they may conflict with those of the present application (in which case the present application prevails).

The present invention provides systems, methods and computer readable media for calibrating signals read from CGH arrays to facilitate more reliable and accurate identification of signals that represent relative DNA copy number differences, such as caused by genetic anomalies (e.g., amplifications and deletions) in the samples represented by the signals read. When one or more calibration arrays are used to determine the noise level on log ratio data read from a CGH array for further processing to determine indications of genetic anomalies, and the one or more calibration arrays used are CGH arrays having only reference samples contacted thereto, the standard deviation of the signals from such calibration arrays may be used for calculating a noise level for those calibration arrays, which may then be used as a measure of noise when processing an array having a test sample contacted thereto.

One problem with this approach is that the estimation of noise from a calibration or "normal" array may often underestimate the noise of the log ratio signals for a sample CGH array containing a sample that does exhibit genetic anomalies, because when some or all of the chromosomes on the sample array show anomalous behavior, the amplified or deleted regions of those chromosomes also contribute to the calculation of noise. Thus, better results may be obtained by calculating the log ratio noise directly from the sample array containing the sample that is being examined for the presence of genetic anomalies.

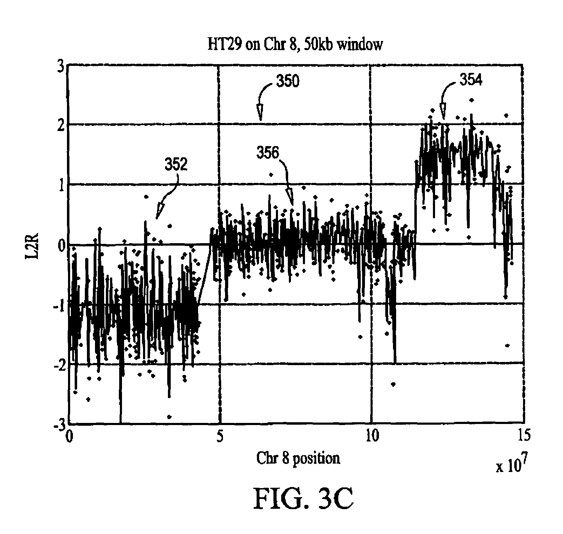

Also, when processing CGH arrays, it has been observed that the use of standard deviation of the log ratio signals to compute a noise estimate, may not estimate noise very well for CGH array applications, particularly in areas where duplication or amplification is reported. That is, it has been observed that noise levels for probes representative of chromosome locations where amplification or deletion has occurred have been observed to be higher than noise levels of probes representative of chromosome locations that are normal (i.e., no amplification or deletion occurring). Figs. 3C-3D illustrate examples of these differences in noise levels, and are explained in more detail below.

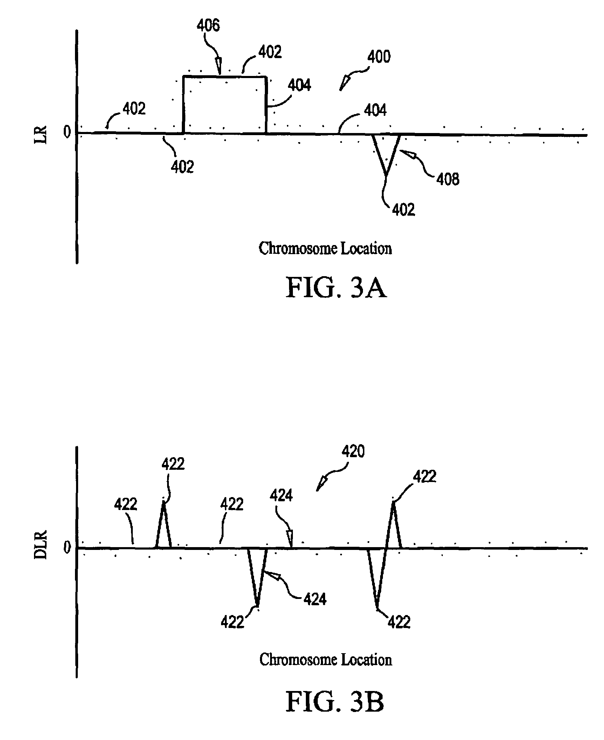

A more robust estimate of noise characterizing a CGH array may be outputted by calculating the spread of the log ratio differences between consecutive probes along all chromosomes represented by the CGH array, divided by the square root of 2 to counteract the effect of noise averaging. Referring to Fig. 3A, an illustration of a plot 400 of log ratio values between two channels from a CGH array is provided. The probe signals from the array have been rearranged to correlate to their positions represented on the chromosomes in the plot 400, thereby mapping them to the chromosome locations (chromosomal coordinates represented by each probe, respectively), with log ratio values of the two channels for each location/probe represented by data points 402. That is, the probes, as arranged, are capable of hybridizing, under stringent conditions, to consecutive positions along a chromosome. Note that consecutive does not necessarily mean directly adjacent to, as consecutive arrangement is defined along a consistent direction, e.g., such as from one chromosome arm to another along the same chromosome. An average log ratio value line 404 has been drawn based upon the plotted points 402. Where the genetic material is "normal" and no amplification or deletion has been reported, the average log ratio signal is about zero, as shown, and is expected, since the fold number should be the same in both channels. When one channel represents abnormal tissue, such as cancer tissue, for example, and the other channel is a control channel representing normal or non-cancerous tissue, then the regions in which amplification or deletion has occurred in the cancerous or otherwise abnormal tissue shows up by log ratio values that deviate from zero, e.g., a value around +1 for an amplification of two, such as the amplification region 406 shown in Fig. 3A or a significantly negative value indicating a deletion, such as illustrated in region 408 in Fig. 3A. The amount of the negative value plotted depends on the average ploidy of the sample and the copy number of the deletion or amplification. For example, if the average ploidy is two, a 1:2 deletion will show a log ratio of about -0.7 to about -1.0. As another example, where the average ploidy is two, a 3:2 amplification (i.e., where the copy number in the abnormal tissue sample is three and the copy number of the normal tissue sample is 2) will show a log ratio of about 0.4 to about 0.6.

Even highly aneuploid samples have extensive stretches of many chromosomes along which the genomic copy number is constant, or nearly so. In such constant copy number regions, the true log ratios are constant, though not necessarily zero. The "eyeball" judgment of log ratio error, based on a user's visualization of a plot of log ratio values plotted in accordance with the locations of probes relative to the chromosomal locations that they are representing, and hence the judgment of the minimum log ratio difference required to make reliable amplification or deletion calls, is based on observing the variation in such constant copy number regions. The DLRSpread (derivative of log ratio spread) metric is provided for automated quantitation of this type of judgment.

For normal samples, the DLRSpread metric is just the width (standard deviation of the distribution) of a self-self distribution, i.e., an array having the same sample bonded to probes as read by both channels of a two channel analyzer, such as a scanner. This width is generally below about 0.2 $\log_2$ units of the plot, and closely approximates the spread of the log ratio signals in a calibration array. For an array measuring genetic anomalies exhibited in one or more chromosomes of a sample, however, the width will be somewhat greater, because the width of regions of constant copy number different from two will include both noise and the variable log ratio compression observed form many probes, and regions that may appear to be constant copy number regions may actually have single probes or small regions in which the copy number in fact varies.

Fig. 3B illustrates a plot 420 of the derivatives of the log ratio values plotted in Fig. 3A. The derivative values are calculated by $DLR_i = LR_i - LR_{i-1}$, where DLRi is the derivative of the log ratio value at log ratio data point i, $LR_i$ is the log ratio value data point i and $LR_{i-1}$ is the i-1$^{st}$ log ratio data point, and i ranges from one to the total number of data points. The derivative values DLR are all around zero value on average, as indicated by average value line 424 plotted based upon DLR values 422, except for values that initially jump or drop to begin or end the indication of an amplification or deletion.

To make the DLRSpread metric more robust and a truer measure of noise, an inter-quartile range of the DLR value 422 may be determined to get rid of the outliers (with appropriate scaling to transform the distribution to a normal distribution, e.g., by ranking the DLR values 422 from lowest to highest or highest to lowest, and then considering only those data points from the twenty-fifth percentile to the seventy-fifth percentile of the ranked range), to eliminate the outlier values, including those defining the spikes in the plot of Fig. 3B, to calculate the spread of the distribution, rather than directly calculating the standard deviation of the derivative of log ratio signals.

The DLR values in the inter-quartile range, referred to as DIQR are then mathematically converted according to the following formula to provide the spread of the derivative of log ratio values: $Spread = (dIQR/erfinv(0.5)*2*\sqrt{2}/\sqrt{2})$. The extra $\sqrt{2}$ division is needed to convert from derivative log ratio space to log ratio space, and $erfinv(0.5)*2*\sqrt{2}$ is a constant (roughly equal to about 1.349) that compensates for use of the inter-quartile range as opposed to the entire range, based upon a normal distribution, where "erfinv" represents an inverse error function. Thus the spread of the derivative of log ratio values is determined as a measure of noise characterizing the array.

Fig. 3C illustrates the phenomenon described above, wherein it has been observed that noise levels for probes representative of chromosome locations where amplification or deletion has occurred have been observed to be higher than noise levels of probes representative of chromosome locations that are normal (i.e., no amplification or deletion occurring). A plot of the $\log_2$ standard deviation of noise for chromosome 8 in the ht29 cancer cell line is shown 350 in a 50 Kb window. The portion displayed has distinct regions of copy number loss 352 and copy number gain 354 relative to the region 356 showing normal copy number. It can be readily visually observed that the regions 352, 354 with copy numbers differing from the average ploidy of the genome are noisier than region 356 having the average ploidy, as deviation from a median value in those regions is greater than in region 356. Thus, the noise may be different in different locations of a sample measured, as noted, due to variations in ploidy. Using derivative log ratio noise calculation techniques described herein, the system identifies the ploidy of different parts of the genome and calculates the noise for those sections separately, rather than using one noise estimate for the entire array.

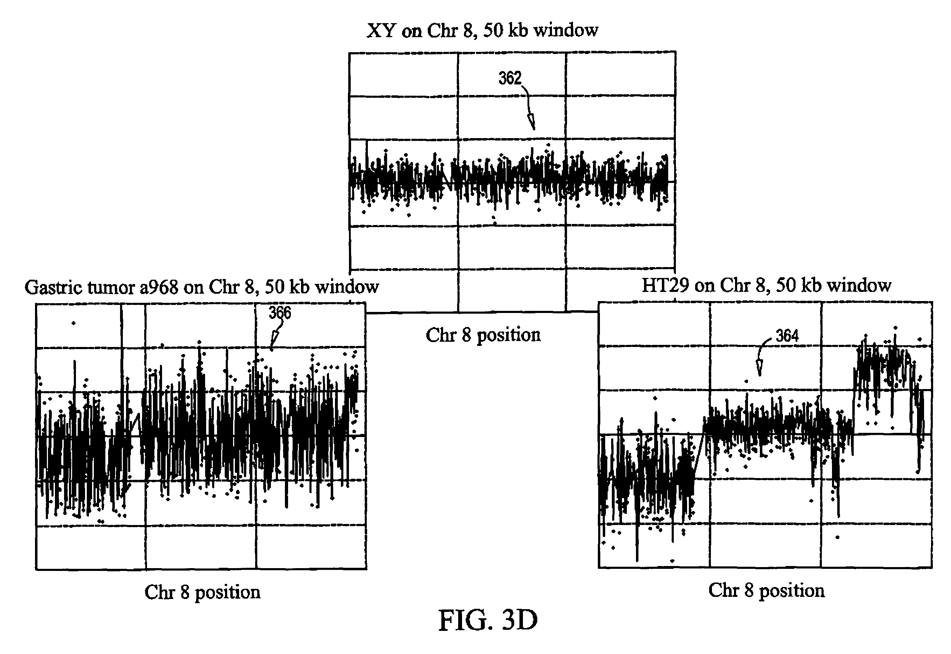

Fig. 3D is a display of charts comparing noise levels among diploid cell lines having been plotted 362 with noise levels associated with aneuploid cell lines having been plotted 364 and tumor cell lines 366. The $\log_2$ standard deviation of noise plotted with regard to the diploid cell lines 362 is less than about 0.2, while $\log_2$ standard deviation of noise plotted with regard to the aneuploid cell lines (HT29) is about 0.3 and $\log_2$ standard deviation of noise plotted with regard to the tumor cell lines 366 is greater still.

The DLR metric can be calculated from the log ratios of signals from a calibration array or from the log ratios of signals from a sample array (e.g., wherein a sample array compares a "normal" tissue sample to an "abnormal" tissue sample expected to exhibit genetic anomalies). In either case, the DLR metric can be used for further statistical analysis of aberrant chromosomal regions to determine whether and where anomalies/aberrations (e.g., amplifications, deletions) exist in the signal data obtained from a sample array. One fast method of statistically analyzing aberrant regions is based on hypergeometric Z-scores, and can be used for a quick initial identification of "statistically interesting" chromosomal regions, i.e., regions of chromosomes from which signal data representative of those regions has been determined by hypergeometric Z-scoring to likely contain one or more aberrations. Once a statistically interesting region or chromosome has been identified, a more detailed search for aberrant regions may be carried out. One example of an algorithm for a more detailed search is described below.

A goal is to identify regions of a chromosome from which the data considered indicates that these regions may contain differences in copy number compared to a reference sample, such as resulting from genetic aberrations, for example. One way in which it may be decided to identify a probe (signals from a probe) as indicating an aberration in the region of a chromosome for which that probe is designed, is that it is determined to be statistically far away from the mean value of the signals (e.g., zero), and is statistically determined to be far enough away from the mean that it is unlikely that this value is the result of a random occurrence (i.e., an outlier). By assigning a p-value to each data point (e.g., signal read from probe) that is distant from the mean value, the p-value gives the probability that the occurrence is due to chance alone (i.e., random occurrence). Thus, the lower the p-value is, the more confidence there is that the data point being considered is actually due to an aberration.

One way of making determinations as to likely aberrations is to assume that the entire log ratio distribution of signals (test sample signals to respective reference sample signals) is completely due to noise, is centered as zero, and varies about zero by some standard deviation, and that the noise distribution is normal (Gaussian). By calculating the standard deviation (width) of the assumed Gaussian distribution of the log ratios of the signals, the p-values can be calculated based upon the calculated standard deviation according to known techniques.

However, it has been found that although the noise distribution for this type of data set is mostly Gaussian, the actual distribution deviates from the normal (Gaussian) distribution mainly at the two tails of the distribution. Although these deviations at the tails generally make up only a small number of data points (from probes), it is important to consider these data points as they are more likely to represent aberrations. The additional data points in the tails have the effect of overestimating the value of the standard deviation when calculated based on an assumption of a Gaussian distribution of the data points. Accordingly, the present approaches calculate a spread of the distribution (DLRSpread) rather than a standard deviation, where DLRSpread = IQR/2 $\sqrt{2}$ erfinv(.5), where DLRSpread is the spread, IQR is the inter-quartile range of the distribution after proper scaling, erf is the error function of the scaled distribution, defined as the integral of that distribution, and erfinv is the inverse of the error function.

The spread estimates what the standard deviation of the distribution would be if the anomalies at the tails were ignored. The inter-quartile range of the distribution (with proper scaling to transform the distribution to a Gaussian distribution) is used to calculate the spread. If the distribution of the data points were truly Gaussian, then the standard deviation is defined by sd = IQR/2 $\sqrt{2}$ erfinv(.5), where sd is the standard deviation, IQR is defined in the preceding paragraph, erf is the error function, defined as the integral of the Gaussian distribution, and erfinv is defined in the preceding paragraph. The spread equals the standard deviation of a distribution which is truly Gaussian.

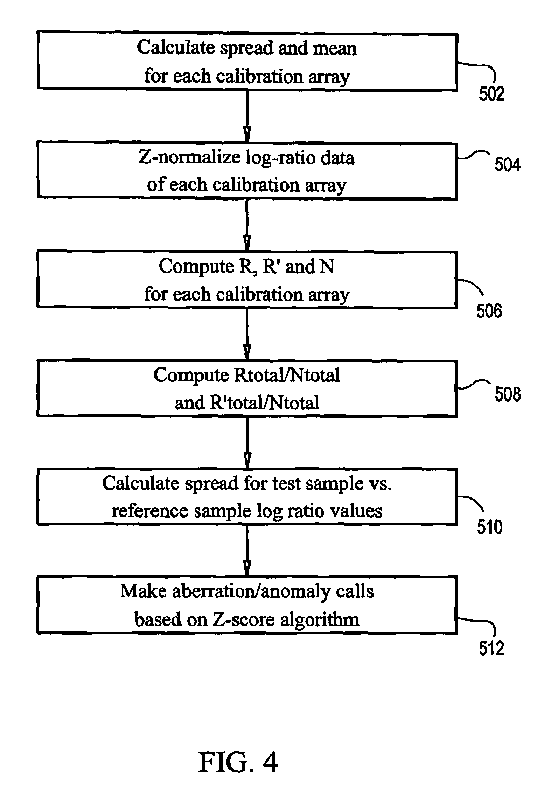

The Z-scoring algorithm is applied to assess the data points in the tails of the distribution to identify those data points which are considered to be true aberrations versus those data points that are identified to be due to noise (outliers). Various different approaches are provided for applying a Z-scoring algorithm to identify potential aberrations in log ratio data taken from locations along a chromosome in a test sample and the same locations along a chromosome in a reference sample. If one or more calibration arrays are to be used in an analysis, then, at event 502 (Fig. 4) the spread is calculated for each calibration array to be used, as well as the mean (mean log ratio value) for each calibration array, each of which should be close to zero.

For each calibration array, the log ratio values may be Z-normalized for hypergeometric Z-scoring at event 504, according to the formula:

$$Z(x) = \frac{x - \mu}{\sigma} \qquad\qquad (1)$$

where

$x$ is the log value of a measured CGH ratio,

$\mu$ is the mean of the log values of all measured CGH ratios, and

σ is the standard deviation of log values of all measured CGH ratios.

The values μ and σ may be calculated from the current calibration array being considered, or from all calibration arrays considered. In either case, chromosomes X and Y are not included in the calculation of μ and σ since gender differences between the samples on different calibration arrays may offset the statistics even for arrays that were designed to be calibration arrays.

Each Z-normalized score can be classified as significantly above or below the mean by using a Z cutoff value ($Z_c$). This cutoff value may be a user-specified value, and may vary depending upon the context of the experiment/ analysis being conducted. By setting the value of $Z_c$, Z-normalized log ratio values greater than $Z_c$ and Z-normalized log ratio values less than -$Z_c$ are considered to be outliers from the normal population of Z-normalized log ratio values, and hence, those log ratio values corresponding to the Z-normalized values greater than $Z_c$ and less than -$Z_c$ are considered to be outliers from the normal population of log ratio values. It is important to note that $Z_c$ is not a cutoff used to filter data, but is a cutoff used for classifying data as being significantly above or below the mean. Thus, $Z_c$ is referred to as a threshold.

Based upon the threshold $Z_c$, a count of the number of Z-normalized log ratio values in each of three classes, R, R' and N are next computed at event 506, where R= the number of Z-normalized log ratio values greater than $Z_c$, R'= the number of Z-normalized log ratio values less than -$Z_c$, and N = the total number of log ratio values. The Z-normalized values and counts can be reserved for subsequent calculations with regard to analysis of one or more sample arrays, and will only need to be recomputed when a different $Z_c$ value is used. Even when a different $Z_c$ value is used, the values for μ and σ do not need to be recomputed.

When more than one calibration array is used, then $R_{total}$, $R'_{total}$ and $N_{total}$ are computed as the sums of the R, R' and N values from each calibration array, and average R and R' values are computed at event 508 as $R_{total}/N_{tatal}$) and $R'_{total}/N_{total}$, respectively. At event 510, the spread (DLRSpread) is computed for log ratio signal values for respective probe signals from a test sample versus a reference sample and this spread value is used as the spread of log ratio noise in further Z-score analysis at event 512. As noted above, by setting the value of $Z_c$ and -$Z_c$ (i.e., the absolute value of -$Z_c$ does not have to equal $Z_c$ as different thresholds may be set), Z-normalized log ratio values greater than $Z_c$ and Z-normalized log ratio values less than -$Z_c$ are considered to be outliers from the normal population of Z-normalized log ratio values, and hence, those log ratio values corresponding to the Z-normalized values greater than $Z_c$ and less than -$Z_c$ are considered to be outliers from the normal population of log ratio values. For these outliers so identified, the Z-scoring algorithm explicitly takes these outliers into account to make a distinction between those data points which are considered to be truly outliers, versus those data points which are considered to be true aberrations. Thus, the Z-scoring algorithm counts R and R' and computes the probability of a true outlier, without counting true aberrations. In the procedure of Fig. 4, this may be directly calculated from the one or more calibration arrays, as R/N and R'/N.

To calculate the hypergeometric Z-score for individual probes in the test sample, a moving window of predetermined width may be used. The window w may be predefined as a predetermined number of adjacent measurements, or it may have a predetermined length as a positional window, (e.g., every megabase), or it may be a variably sized window. This portion of the analysis involves identifying the over-abundance or under-abundance of log ratio values that deviate significantly from the mean value identified in equation (1) (i.e., μ) and which lie inside window $w$. From this smaller subset of the log ratio data inside the window w, counts r, r' and n are computed in the same manner that R, R' and N were computed in event 506, but where r = the number of log ratio values (z-normalized log ratio values or log ratio values that have not been z-normalized) greater than $Z_c$ and within $w$, r' = the number of log ratio values less than -$Z_c$ and within $w$, and n = the total number of log ratio values within $w$.

Using these computed values, an exact Z-score can be computed that measures the significance of the over-abundance and/or under-abundance in $w$ of significant positive deviations as:

$$Z(w) = \frac{\left(r - n\frac{R}{N}\right)}{\sqrt{n\left(\frac{R}{N}\right)\left(1 - \frac{R}{N}\right)\left(1 - \frac{n-1}{N-1}\right)}} \qquad (2)$$

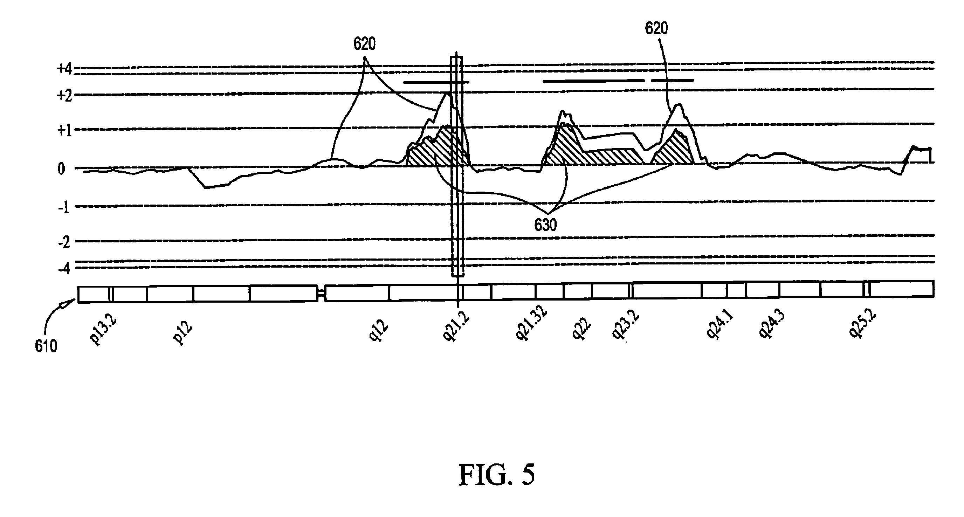

Formula (2) can also be computed for r' to obtain a score for significant negative deviations. These scores identify statistically significant groups of probes that appear to deviate from the typical distribution of log ratio values for the test sample versus reference sample that was analyzed, thus providing a predictive tool to base amplification or deletion event calls on. When these exact Z-scores are computed based on equation (2) above, they may then be plotted similar to a moving average plot as shown in Fig 5. Fig. 5 shows CGH data plotted for Chromosome 17 (Cell Line BT474) 610 relative to the positions on the chromosome 17 shown which the data characterizes. Plot 620 shows the moving average of microarray measurements using a 2 megabase window, and the shaded areas 630 report the hypergeometric Z-score calculated as described herein. Alternatively, the hypergeometric Z-scores can be plotted as a curve like the style of the moving average 620 shown. However, the curve 630 has been space filled in Fig. 5 to make it more readily visually distinguishable from the moving average plot 620.

Another approach to a more detailed examination of statistically interesting regions of chromosomes as indicated by the Z-normalized scoring and analysis above involves finding intervals of consistent high or low log ratios within an ordered (as corresponding to chromosome location) set of probes by measuring a set of genomic locations and considering their genomic order. This technique assigns scores to intervals ($I$) of log ratio signals from the sample array. The scores are designed to reflect the statistical significance of the observed consistency of the high or low log ratio signals, and are useful in several levels of DNA copy number (CDN) data analysis. By using adequately defined statistical scores, significant common aberrations may be identified or called out. Further detailed information about this technique can be found in co-pending, commonly owned Application Serial No. 10/953,958 (U.S. Patent Publication No. 2006/0084067) filed on September 29, 2004 and titled "Method and System for Analysis of Array-Based, Comparative-Hybridization Data. Application Serial No. 10/953,958 and U.S. Patent Publication No. 2006/0084067 are hereby incorporated herein, in their entireties, by reference thereto.

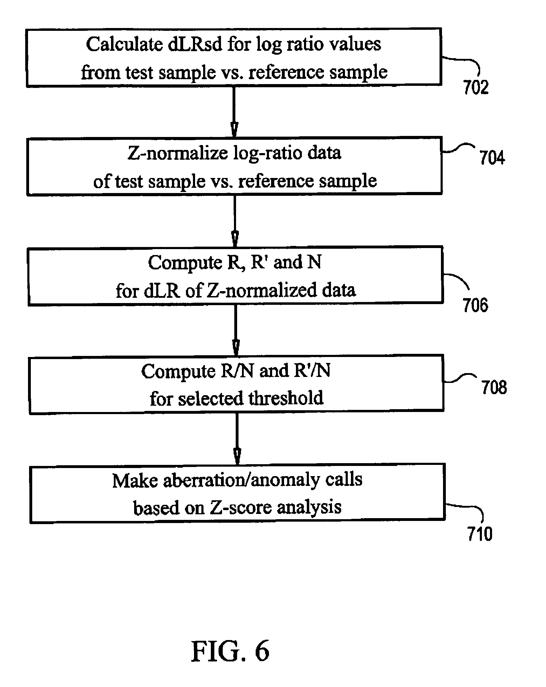

If no calibration arrays are to be used in an analysis, Fig. 6 illustrates events that may be carried out according to one option for analyzing the data, in which the statistics are calculated from the test sample versus reference sample log ratio values. At event 702, the spread of derivative of log ratio values (DLRSpread) of the test sample versus reference sample is calculated. Next, the log ratio values are Z-normalized for hypergeometric Z-scoring at event 704 according to the following:

$$Z(x) = \frac{x - \mu}{spread} \qquad\qquad (5)$$

where
$x$ is the log value of a measured CGH ratio,
$\mu$, the mean is set to its nominal value of zero, and
spread = the calculated DLRSpread, as described above.

The hypergeometric score (i.e., Z-score) assigns a p-value based on the number of outliers compared to the probability of an outlier occurring by random chance. As noted earlier, each z-normalized log ratio value can be classified as significantly above or below the mean by using a Z cutoff value ($Z_c$). This cutoff value may be a user-specified value, and may vary depending upon the context of the experiment/analysis being conducted. By setting the value of $Z_c$, z-normalized log ratio values greater than $Z_c$ and z-normalized log ratio values less than $-Z_c$ are considered to be outliers from the normal population of z-normalized log ratio values, and hence, those log ratio values corresponding to the z-normalized values greater than $Z_c$ and less than $-Z_c$ are considered to be outliers from the normal population of log ratio values. It is important to note that $Z_c$ is not a cutoff used to filter data, but is a cutoff used for classifying data as being significantly above or below the mean. Thus, $Z_c$ is referred to as a threshold.

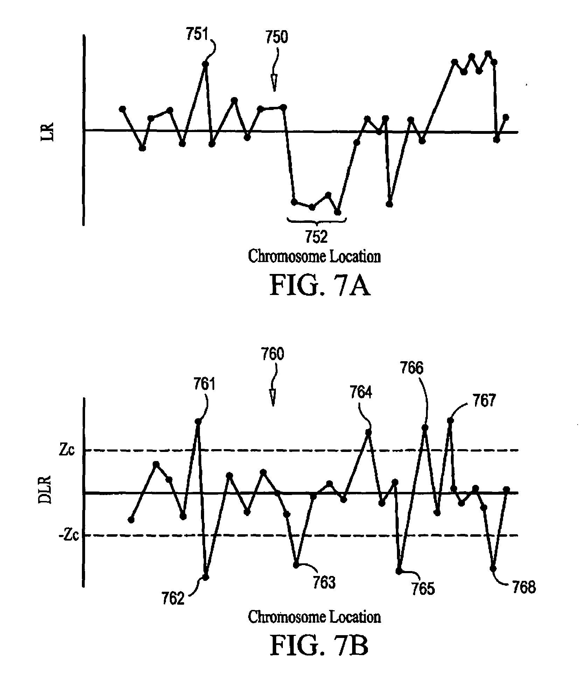

To count R, R' and N when a calibration array is not used, the derivatives of the log ratios (DLR) of the test sample signals to the reference sample signals are considered. Fig. 7A schematically illustrates a plot of log ratio values of test sample signals to reference sample signals against the relative chromosomal positions represented by the signals received. Fig. 7B shows a plot of the DLR values for the log ratio values plotted in Fig. 7A. By using the values of $Z_c$ and $-Z_c$ as thresholds (after appropriate scaling, since the signal is DLR), the system can determine the value of R and R' (and N) by counting the number of data points that exceed the thresholds. For example, in Fig. 7B the data points 761, 762, 763, 764, 765, 766, 767 and 768 can readily be identified as exceeding one or the other of the thresholds $Z_c$ and $-Z_c$. Note that in the derivative scale, the thresholds are also scaled by multiplying by $\sqrt{2}$.

A true outlier data point is likely to occur as a single data point, as the probability of two or more consecutive data points occurring as outliers by pure chance is very low. For example, in a typical case, there may be on the order of one hundred outlier data points from a total population of 50,000 data points; thus, it is very unlikely that two or more true outliers will occur along consecutive locations relative to the chromosome by pure chance. Likewise, genetic anomalies that occur along the chromosome often show a series of adjacent probes generating signals that are in the outlier ranges. Although it is possible to have a single data point (probe) reflecting a genetic anomaly, this is also rare. Also, in such an occurrence, counting this occurrence as a true outlier results in more conservative guidelines for calling out true aberrations, and this is preferable to erring in a way that would be more likely to identify true outliers as aberrations. For single data point outliers, the derivative log ratio values resulting therefrom show consecutive data points in the outlier regions. For example, the data point 751 in Fig. 7A shows DLR values corresponding thereto that first show a spike 761 up into the positive outlier region followed immediately by a spike down 762 to the negative outlier region.

Typical regions of genetic anomaly (i.e., where there is a transition in the data values from a normal copy number to an abnormal copy number) show a series of consecutive data points (probes) that are in the outlier region. For example, the region 752 in Fig. 7A shows 4 consecutive data points (probes) along a region of the chromosome reporting signal values that are far from the mean signal value (zero). The corresponding DLR values show and initial spike 763 downward into the negative outlier values, and then do not show a spike upward 764 (indicating transition from the abnormal copy number back to normal copy number) until the fourth data point after data point 763.

Given the nature of the DLR spikes, the system can be programmed to recognize consecutive spikes (e.g., 761 and 762, 765 and 766 in Fig. 7B) as true outliers, for the reasons described above, whereas single spikes (i.e., those not immediately followed or preceded by a data point having a spiked value in an outlier region) are considered to potentially be real genetic anomalies. Also, due to the ordering of the data points, the system can assign a spike pair characterized by a positive spike followed by a negative spike (e.g., 761,762) to R, as being a positive outlier, and conversely, can assign a spike pair characterized by a negative spike followed by a positive spike (e.g., 765,766) to R', as being a negative outlier. By this technique, the single spikes (e.g., 763, 764, 767 and 768 in Fig. 7B) are excluded from being counted as contributing to R and R'. Alternatively, a simpler counting technique may be carried out where the system simply counts all of the outlying data points and if a counted data point is in the positive direction, it contributes to R, while a counted data point in the negative direction contributes to R'.

Once the number of Z-normalized log ratio values in each of three classes, R, R' and N have been determined at event 706, values for R/N and R'/N for the selected thresholds ($Z_c$ and $-Z_c$) are computed at event 708. At event 710, the hypergeometric Z-score is computed based on the calculated R/N and R'/N values and the selected $Z_c$ and $-Z_c$ thresholds, using the formula described above as equation (2).

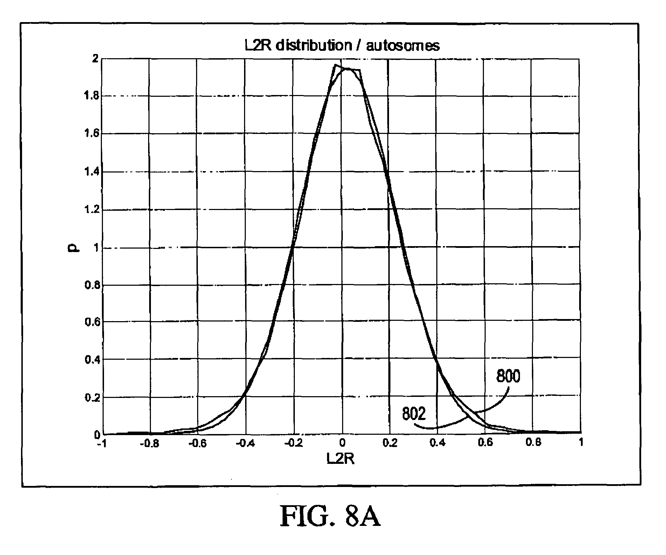

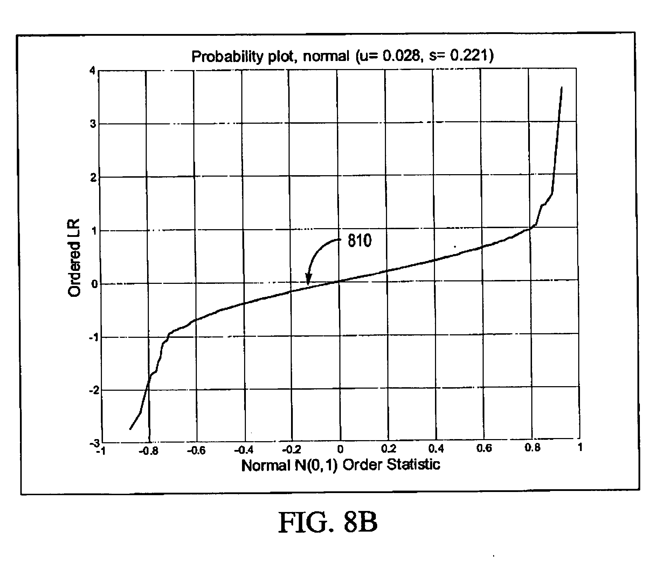

As noted above, the distribution of log ratio values taken from probes hybridized with a test sample versus the same probes hybridized with a reference sample, where the test sample contains chromosomal aberrations, is not truly Gaussian. Although the noise associated with the log ratio data values from an array containing probes to which such a test sample and reference sample have been co-hybridized is primarily normally (Gaussian) distributed, the distribution deviates from the normal distribution, mostly at the two tails of the curve plotting the distribution of the noise. Fig. 8A shows a plot 800 of a distribution of observed log (i.e., $\log_2$) ratio values from autosomes of an individual male/female assay (considered to have no chromosomal aberrations) compared with a plot 802 of a reference normal distribution. Fig. 8B is a probability plot 810 of the observed log ratio values from Fig. 8A versus Gaussian expectations (i.e., the values that are plotted in the Gaussian distribution of Fig. 7A). If the distribution of observed log ratio values from Fig. 8A was truly Gaussian, then plot 810 would be a straight line. However, it can be seen that the distribution deviates from a true normal (Gaussian) distribution, especially at the tail portions of the distribution.

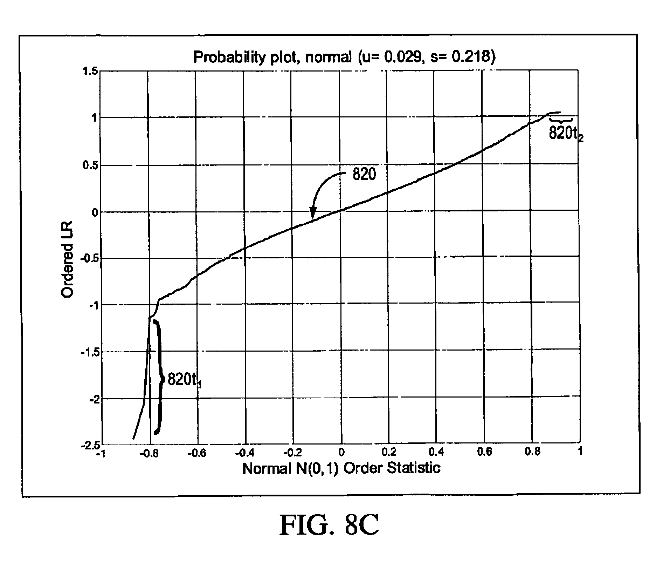

Fig. 8C shows another probability plot 820 of observed log ratio values from a different array from that associated with Figs. 8A and 8B, versus Gaussian expectations. In plot 820, the tail portions 820t1 and 820t2 of the plotted data represented only 20 of the 38220 autosomal probes from which log ratio data were plotted. Thirteen of those twenty were signals from thirteen probes among 132 probes that failed filters applied to the data to ensure that the signals were positively found as originating from a probe, had positive and significant signal levels, had signal levels well above background signal levels, were not saturated, had a background subtracted signal level above a predetermined threshold level, and were not feature population outliers, with regard to both channels of data read. The 5 (of 38088) bad autosomal probes that passed the filters still skewed the distribution, but much less so. The remaining seven (of 38,088) probes that passed the filters still skewed the distribution.

As noted, an objective of the Z-score algorithm (see equation (2)) is to compare the outliers for log ratio values from a normal sample (having no genetic anomalies) to the outliers for log ratio values from an abnormal sample (i.e., containing a genetic anomaly), wherein if the number of outliers from the abnormal sample is much greater for any window of data points than the number of outliers from the normal sample for the corresponding window of data points, than it is concluded that an aberration has occurred in the abnormal sample at the location identified by the middle of the window. When the distribution of noise for the log ratio values from the abnormal sample is assumed to be purely Gaussian, the result may be that more aberration calls are made then what actually exist in the abnormal sample. The techniques described above reduce the number of aberration calls that do not actually identify aberrations (i.e., false positives).

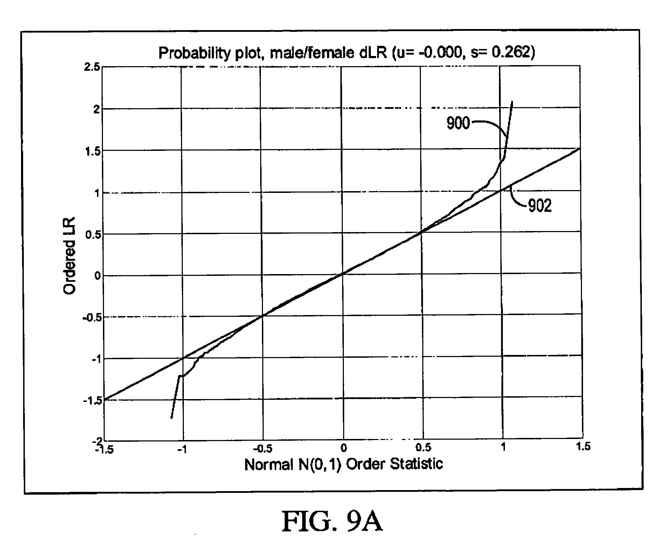

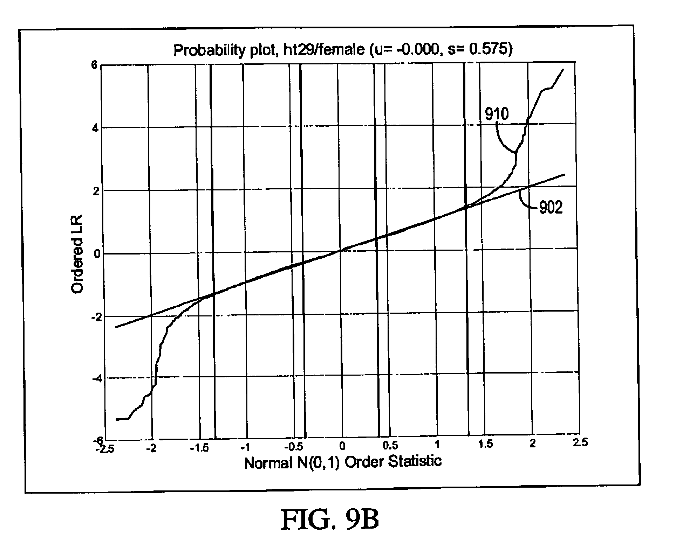

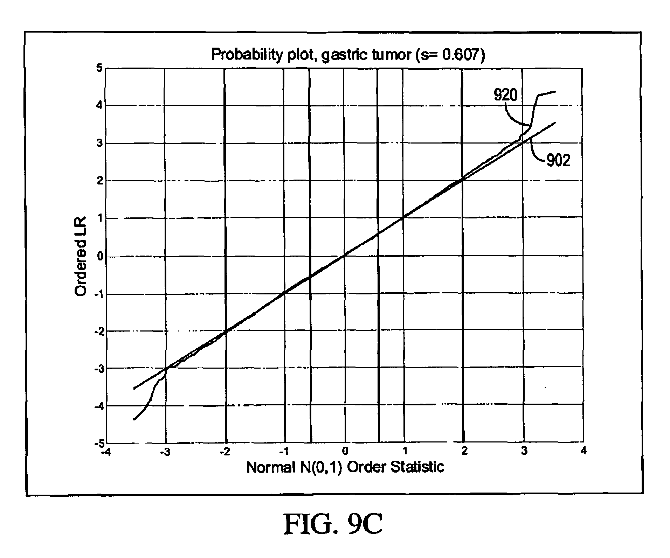

Fig. 9A shows a probability plot 900 of the log ratio values from Fig. 8A versus Gaussian expectations, and thus corresponds to the plot of Fig. 8B except for the statistical processing option where R, R' and N are computed from the derivative of the log ratio (dLR) (after proper normalization) was used to reduce the number of false positives, in the manner described above. Fig. 9B similarly plots a probability plot 910 of observed log ratio values from a sample containing tissue in the ht29 cancer cell line with normal tissue in the reference channel, versus Gaussian expectation, when using the same statistical processing used in Fig. 9A. When compared with the Gaussian expectation plot 902, the plots 900 and 910 are reliably Gaussian for about 98% of the data. The extended tails that deviate from Gaussian distribution included only about three to five bad probes in Fig. 9A, and all of the real copy number changes (i.e., probes, the signals from which were describing aberrations) in Fig. 9B. Even very aberrant tumors may be processed according to this technique to provide a substantially Gaussian distribution of the log ratio signals. Fig. 9C is a plot 920, similar to plots 900 and 910, but where the sample array contained a very aberrant tumor tissue (i.e., a gastric tumor from VTT). Vertical lines in Figs. 9B and 9C mark the $1^{st}$, $25^{th}$, $75^{th}$ and $99^{th}$ percentiles of the plotted log ratio data.

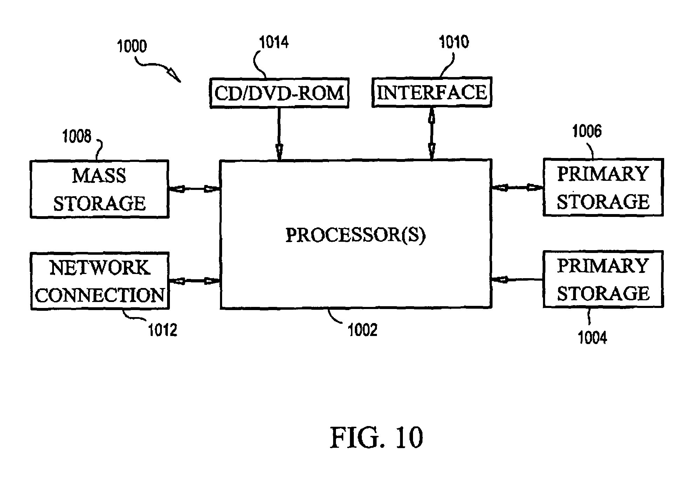

Fig. 10 is a schematic illustration of a typical computer system that may be used to perform procedures described above. The computer system 1000 includes any number of processors 1002 (also referred to as central processing units, or CPUs) that are coupled to storage devices including primary storage 1006 (typically a random access memory, or RAM), primary storage 1004 (typically a read only memory, or ROM). As is well known in the art, primary storage 1004 acts to transfer data and instructions uni-directionally to the CPU and primary storage 1006 is used typically to transfer data and instructions in a bi-directional manner Both of these primary storage devices may include any suitable computer-readable media such as those described above. A mass storage device 1008 is also coupled bi-directionally to CPU 1002 and provides additional data storage capacity and may include any of the computer-readable media described above. Mass storage device 1008 may be used to store programs, data and the like and is typically a secondary storage medium such as a hard disk that is slower than primary storage. It will be appreciated that the information retained within the mass storage device 1008, may, in appropriate cases, be incorporated in standard fashion as part of primary storage 1006 as virtual memory. A specific mass storage device such as a CD-ROM or DVD-ROM 1014 may also pass data uni-directionally to the CPU.

CPU 1002 is also coupled to an interface 1010 that includes one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 1002 optionally may be coupled to a computer or telecommunications network using a network connection as shown generally at 1012. With such a network connection, it is contemplated that the CPU might receive information from the network, or might output information to the network in the course of performing the above-described method steps. The above-described devices and materials will be familiar to those of skill in the computer hardware and software arts.

The hardware elements described above may implement the instructions of multiple software modules for performing the operations of this invention. For example, instructions for calculating the derivative log ratio spread of signals red from an array may be stored on mass storage device 1008 or 1014 and executed on CPU 1008 in conjunction with primary memory 1006.

In addition, embodiments of the present invention further relate to computer readable media or computer program products that include program instructions and/or data (including data structures) for performing various computer-implemented operations. The media and program instructions may be those specially designed and constructed for the purposes of the present invention, or they may be of the kind well known and available to those having skill in the computer software arts. Examples of computer-readable media include, but are not limited to, magnetic media such as hard disks, floppy disks, and magnetic tape; optical media such as CD-ROM, CDRW, DVD-ROM, or DVD-RW disks; magneto-optical media such as floptical disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, and scope of the present invention. All such modifications are intended to be within the scope of the claims appended hereto.

The disclosures in United States patent application no. 11/545,962, from which this application claims priority, and in the abstract accompanying this application is incorporated herein by reference.

**Claims**

1. A method for calling out genetic aberrations, said method comprising:

   estimating log ratio noise associated with log ratio signals read from respective probes on at least one array for signals representative of the same chromosomal locations in a test sample of nucleic acids and a reference sample of nucleic acids applied to said at least one array; and
   comparing outliers for log ratio values from the reference sample to outliers for log ratio values from the test sample; and
   outputting a copy number of one or more of said chromosomal locations in the test sample relative to the reference sample for viewing by a user.

**2.** The method of claim 1, wherein said comparing comprises comparing outliers for log ratio values from the reference tissue for data points defined by a window extending along a chromosomal location that the data points correspond to, to the data points from the test sample defined by the window in the same corresponding chromosomal location regarding the test sample data points.

**3.** The method of claim 1 or 2, wherein said estimating comprises calculating the spread of log ratio noise directly from signals from probes contacted to said test sample and signals from probes contacted to said reference sample (702).

**4.** The method of claim 1 or 2, wherein said estimating comprises calculating the spread of log ratio noise from signals from probes on at least one calibration array (502).

**5.** The method of claim 4, wherein said calculating comprises:

Z-normalizing log ratio signal values from said at least one calibration array (504); and
setting positive and negative Z-cutoff values.

**6.** The method of claim 5, wherein said outliers are identified by Z-normalized values greater than said positive Z-cutoff value and Z-normalized values less than said negative Z-cutoff value.

**7.** The method of claim 5 or 6, further comprising calculating the spread of log ratio signals read from respective probes for signals representative of respective chromosomal locations in the test sample and reference sample (510).

**8.** The method of any of claims 5 to 7, further comprising:

providing a window that surrounds a subset of the log ratio signal values from the probes contacted with the test sample versus the probes contacted with the reference sample;
identifying overabundance or under-abundance of log ratio values that exceed the positive Z-cutoff value or negative Z-cutoff value, respectively; compared to the of log ratio values from the at least one calibration array that exceed the positive Z-cutoff value or negative Z-cutoff value.

**9.** The method of claim 8, further comprising determining whether a positive copy number difference exists between the test and reference sample from Z-scoring according to:

$$Z(w) = \frac{\left(r - n\frac{R}{N}\right)}{\sqrt{n\left(\frac{R}{N}\right)\left(1 - \frac{R}{N}\right)\left(1 - \frac{n-1}{N-1}\right)}}$$

where
$Z(w)$ = the Z-score of log ratio values contained within window w;
$R$ = the number of outliers in the at least one calibration array that exceed the positive cutoff threshold;
$N$ = the total number of log ratio values considered from the at least one calibration array;
$r$ = the number of outliers in the window w that exceed the positive cutoff threshold; and
$n$ = the total number of log ratio values within window w.

**10.** The method of claim 9, further comprising plotting said Z-scores.

**11.** The method of any of claims 1 to 3, wherein said calculating further comprises:

Z-normalizing log ratio signal values from said probes contacted to said test and reference samples (704);
calculating the derivatives of the Z-normalized log ratio signal values (706); and
setting positive and negative Z-cutoff values.

**12.** The method of claim 11, wherein said outliers are identified by Z-normalized derivative log ratio values greater than said positive Z-cutoff value and Z-normalized values less than said negative Z-cutoff value.

13. The method of claim 12, wherein said outliers are identified by pairs of consecutive Z-normalized derivative log ratio values greater than said positive Z-cutoff value or less than said negative Z-cutoff value.

14. The method of claim 12 or 13, further comprising:

providing a window that surrounds a subset of the log ratio signal values from said probes contacted with the test sample versus said probes contacted with the reference sample;
identifying overabundance or under-abundance of log ratio values that exceed the positive Z-cutoff value or negative Z-cutoff value, respectively; and
comparing the overabundance or under-abundance as a percentage of the total number of log ratio signal values within the window, to the number of outliers identified as a percentage of the total number of log ratio signal values.

15. The method of any of claims 11 to 14, further comprising determining whether a positive copy number difference exists between the test and reference samples from Z-scoring (710) according to:

$$Z(w) = \frac{\left(r - n\dfrac{R}{N}\right)}{\sqrt{n\left(\dfrac{R}{N}\right)\left(1 - \dfrac{R}{N}\right)\left(1 - \dfrac{n-1}{N-1}\right)}}$$

where
$Z(w)$ = the Z-score of log ratio values contained within window $w$;
$R$ = the number of outliers identified that exceed the positive cutoff threshold;
$N$ = the total number of log ratio values from the test sample and reference sample tissues;
$r$ = the number of log ratio values in the window $w$ that exceed the positive cutoff threshold; and
$n$ = the total number of log ratio values within window $w$.

16. The method of claim 15, further comprising plotting said Z-scores.

17. A system for calling out genetic aberrations, said system comprising:

at least one processor (1002);
programming, that when executed by said at least one processor carries out the following steps: estimating log ratio noise associated with log ratio signals read from respective probes on at least one array for signals representative of the same chromosomal locations in a test sample of nucleic acids and a reference sample of nucleic acids applied to said at least one array, and comparing outliers for log ratio values from the reference sample to outliers for log ratio values from the test sample; and
an interface (1010) for outputting results for use by a user.

18. The system of claim 17, wherein said at least one processor (1002) executing said programming compares outliers for log ratio values from the reference tissue for data points defined by a window extending along a chromosomal location that the data points correspond to, to the data points from the test sample defined by the window in the same corresponding chromosomal location regarding the test sample data points.

19. The system of claim 17 or 18, wherein said wherein said estimating comprises calculating the spread of log ratio noise directly from signals from probes contacted to said test sample and signals from probes contacted to said reference sample.

20. The system of claim 17 or 18, wherein said wherein said estimating comprises calculating the spread of log ratio noise from signals from probes on at least one calibration array.

21. A computer readable medium carrying one or more sequences of instructions for calling out genetic aberrations, wherein execution of one or more sequences of instructions by one or more processors causes the one or more processors to perform the steps of:

estimating log ratio noise associated with log ratio signals read from respective probes on at least one array for signals representative of the same chromosomal locations in a test sample of nucleic acids and a reference sample of nucleic acids applied to said at least one array; and

comparing outliers for log ratio values from the reference sample to outliers for log ratio values from the test sample; and

outputting a copy number of one or more of said chromosomal locations in the test sample relative to the reference sample for viewing by a user.

22. The computer readable medium of claim 21 wherein execution of one or more sequences of instructions by one or more processors causes the one or more processors to compare outliers for log ratio values from the reference tissue for data points defined by a window extending along a chromosomal location that the data points correspond to, to the data points from the test sample defined by the window in the same corresponding chromosomal location regarding the test sample data points.

23. The computer readable medium of claim 21 or 22, wherein said estimating comprises said estimating comprises calculating the spread of log ratio noise directly from signals from probes contacted to said test sample and signals from probes contacted to said reference sample.

24. The computer readable medium of claim 21 or 22, wherein said estimating comprises calculating the spread of log ratio noise from signals from probes on at least one calibration array.

113c  112  113a

111b

110

111a  113b  113d

## FIG. 1

116  117

116  117

116  116

116  112

## FIG. 2

FIG. 3A

FIG. 3B

FIG. 3C

FIG. 3D

```
┌─────────────────────────────────────┐
│      Calculate spread and mean       │
│       for each calibration array     │──╮ 502
└─────────────────────────────────────┘
                   │
                   ▼
┌─────────────────────────────────────┐
│       Z-normalize log-ratio data     │──╮ 504
│        of each calibration array     │
└─────────────────────────────────────┘
                   │
                   ▼
┌─────────────────────────────────────┐
│        Compute R, R' and N           │──╮ 506
│       for each calibration array     │
└─────────────────────────────────────┘
                   │
                   ▼
┌─────────────────────────────────────┐
│         Compute Rtotal/Ntotal        │──╮ 508
│          and R'total/Ntotal          │
└─────────────────────────────────────┘
                   │
                   ▼
┌─────────────────────────────────────┐
│    Calculate spread for test sample vs.  │──╮ 510
│     reference sample log ratio values    │
└─────────────────────────────────────┘
                   │
                   ▼
┌─────────────────────────────────────┐
│     Make aberration/anomaly calls    │──╮ 512
│       based on Z-score algorithm     │
└─────────────────────────────────────┘
```

FIG. 4

24

FIG. 5

EP 1 939 778 A2

Calculate dLRsd for log ratio values
from test sample vs. reference sample — 702

Z-normalize log-ratio data
of test sample vs. reference sample — 704

Compute R, R' and N
for dLR of Z-normalized data — 706

Compute R/N and R'/N
for selected threshold — 708

Make aberration/anomaly calls
based on Z-score analysis — 710

# FIG. 6

FIG. 7A

FIG. 7B

FIG. 8A

FIG. 8B

FIG. 8C

FIG. 9A

Probability plot, ht29/female (u= -0.000, s= 0.575)

FIG. 9B

FIG. 9C

FIG. 10

EP 1 939 778 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 10744495 A
- US 6410243 B
- WO 9318186 A
- US 6242266 B
- US 6232072 B
- US 6180351 B
- US 6171797 B
- US 6323043 B
- US 6518556 B
- US 6486457 B

- US 6406849 B
- US 6371370 B
- US 6355921 B
- US 6320196 B
- US 6251685 B
- US 6222664 B
- US 6221583 B
- US 10953958 B
- US 20060084067 A
- US 11545962 B